# EUROPEAN PATENT SPECIFICATION

(11) **EP 0 983 262 B1**
(45) Date of publication and mention of the grant of the patent: **09.07.2003**
(21) Application number: 98932069.2
(22) Date of filing: 18.05.1998
(51) Int. Cl.: C07D 401/06, A61K 31/47, C07D 215/52, C07D 491/10

(54) **QUINOLINE-4-CARBOXAMIDE DERIVATIVES AS NK-2 AND NK-3 RECEPTOR ANTAGONISTS**
CHINOLIN-4-CARBOXAMIDDERIVATE ALS NK-2 UND NK-3 REZEPTOR ANTAGONISTEN
DERIVES DE QUINOLINE-4-CARBOXAMIDE COMME ANTAGONISTES DES RECEPTEURS NK-2 ET NK-3

(30) Priority: 23.05.1997 GB 9710750; 17.10.1997 IT MI972354; 16.12.1997 IT MI972775
(43) Date of publication of application: 08.03.2000
(73) Proprietor: GlaxoSmithKline S.p.A., 37135 Verona (IT)
(72) Inventor: GIARDINA, Giuseppe, Arnaldo, Maria, I-20021 Baranzate di Bollate (IT); GRUGNI, Mario, I-20021 Baranzate di Bollate (IT); GRAZIANI, Davide, I-20021 Baranzate di Bollate (IT); RAVEGLIA, Luca, Francesco, I-20021 Baranzate di Bollate (IT)
(74) Representative: Rutter, Keith, Dr.
(86) International application number: EP9803014
(87) International publication number: WO98052942

(56) References cited:
- WO-A-95/32948
- WO-A-97/19926

## Description

The present invention relates to novel compounds, in particular to novel quinoline derivatives, to processes for the preparation of such compounds, to pharmaceutical compositions containing such compounds and to the use of such compounds in medicine.

The mammalian peptide Neurokinin B (NKB) belongs to the Tachykinin (TK) peptide family which also include Substance P (SP) and Neurokinin A (NKA). Pharmacological and molecular biological evidence has shown the existence of three subtypes of TK receptor (NK₁, NK₂ and NK₃) and NKB binds preferentially to the NK₃ receptor although it also recognises the other two receptors with lower affinity (Maggi et al, **1993,** *J. Auton. Pharmacol., 13,* 23-93).

Selective peptidic NK₃ receptor antagonists are known (Drapeau, **1990** *Regul. Pept.,* 31, 125-135), and findings with peptidic NK₃ receptor agonists suggest that NKB, by activating the NK₃ receptor, has a key role in the modulation of neural input in airways, skin, spinal cord and nigro-striatal pathways (Myers and Undem, 1993, *J.Physiol*., 470, 665-679; Counture et al., 1993, *Regul. Peptides,* 46, 426-429; Mccarson and Krause, 1994, *J. Neurosci.,* 14 (2), 712-720; Arenas et al. 1991, *J.Neurosci*., 11, 2332-8). However, the peptide-like nature of the known antagonists makes them likely to be too labile from a metabolic point of view to serve as practical therapeutic agents.

International Applications WO 97/19926 (published 05.06.97) and WO 95/32948 (published 07.12.95) disclose quinoline carboxamides and their use as neurokinin receptor antagonists.

We have now discovered a novel class of non-peptide NK-3 antagonists which are far more stable from a metabolic point of view than the known peptidic NK-3 receptor antagonists and are of potential therapeutic utility. These compounds also have NK-2 antagonist activity and are therefore considered to be of potential use in the prevention and treatment of a wide variety of clinical conditions which are characterized by overstimulation of the tachykinin receptors, in particular NK-3 and NK-2.

These conditions include respiratory diseases, such as chronic obstructive pulmonary disease (COPD), asthma, airway hyperreactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease and urinary incontinence; renal disorders and disorders of the bladder function, (hereinafter referred to as the 'Primary Conditions').

Certain of these compounds also show CNS activity and hence are considered to be of particular use in the treatment of disorders of the central nervous system such as anxiety, depression, psychosis and schizophrenia; neurodegenerative disorders such as AIDS related dementia, senile dementia of the Alzheimer type, Alzheimer's disease, Down's syndrome, Huntington's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilation and vasospastic diseases such as angina, migraine and Reynaud's disease and pain or nociception, for example, that is attributable to or associated with any of the foregoing conditions especially the transmission of pain in migraine, (hereinafter referred to as the 'Secondary Conditions').

The compounds of formula (I) are also considered to be useful as diagnostic tools for assessing the degree to which neurokinin-3 and neurokinin-2 receptor activity (normal, overactivity or underactivity) is implicated in a patient's symptoms.

According to the present invention there is provided a compound, or a solvate or a salt thereof, of formula (I): 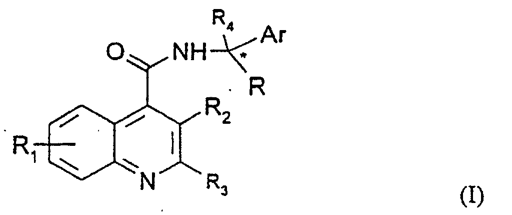 <wherein, R is ethyl, R₁ is H, R₃ is phenyl, R₄ is H, Ar is phenyl and R₂ is selected from the following substituents:

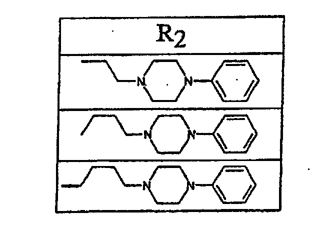

The compounds of formula (I) may have at least one asymmetric centre - for example the carbon atom labelled with an asterisk (*) in the compound of formula (I) - and therefore may exist in more than one stereoisomeric form. The invention extends to all such stereoisomeric forms and to mixtures thereof, including racemates. In particular, the invention includes compounds wherein the asterisked carbon atom in formula (I) has the stereochemistry shown in formula (Ia): 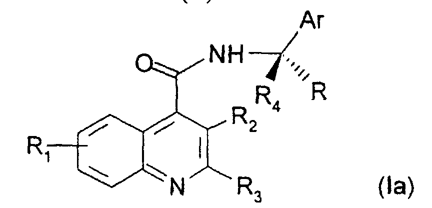 wherein Ar, R, R₁, R₂, R₃, and R₄ are as defined in relation to formula (I).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels.

A substantially pure form will generally contain at least 50% (excluding normal pharmaceutical additives), preferably 75%, more preferably 90% and still more preferably 95% of the compound of formula (I) or its salt or solvate.

One preferred pharmaceutically acceptable form is the crystalline form, including such form in pharmaceutical composition. In the case of salts and solvates the additional ionic and solvent moieties must also be non-toxic.

Suitable salts are pharmaceutically acceptable salts.

Suitable pharmaceutically acceptable salts include the acid addition salts with the conventional pharmaceutical acids, for example maleic, hydrochloric, hydrobromic, phosphoric, acetic, fumaric, salicylic, citric, lactic, mandelic, tartaric, succinic, benzoic, ascorbic and methanesulphonic.

Suitable pharmaceutically acceptable salts include salts of acidic moieties of the compounds of formula (I) when they are present, for example salts of carboxy groups or phenolic hydroxy groups.

Suitable salts of acidic moieties include metal salts, such as for example aluminium, alkali metal salts such as lithium, sodium or potassium, alkaline earth metal salts such as calcium or magnesium and ammonium or substituted ammonium salts, for example those with lower alkylamines such as triethylamine, hydroxy alkylamihes such as 2-hydroxyethylamine, bis-(2-hydroxyethyl)-amine or tri-(2-hydroxyethyl)-amine, cycloalkylamines such as bicyclohexylamine, or with procaine, dibenzylpiperidine, N-benzyl-β-phenethylamine, dehydroabietylamine, N,N'-bisdehydroabietylamine, glucamine, N-methylglucamine or bases of the pyridine type such as pyridine, collidine, quinine or quinoline.

Suitable solvates are pharmaceutically acceptable solvates.

Suitable pharmaceutically acceptable solvates include hydrates.

The invention also provides a process for the preparation of a compound of formula (I), or a salt thereof and/or a solvate thereof, which process comprises reacting a compound of formula (II) or an active derivative thereof: 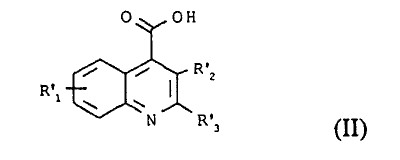 wherein R'₁, R'₂ and R'₃ are R₁, R₂ and R₃ respectively as defined in relation to formula (I) or a group convertible to R₁, R₂ and R₃ respectively; with a compound of formula (III): 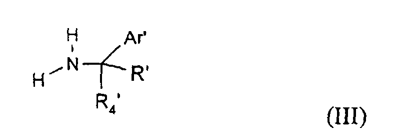 wherein R', R₄' and Ar' are R, R₄ and Ar as defined for formula (I) or a group or atom convertible to R, R₄ and Ar respectively; to form a compound of formula (Ib): 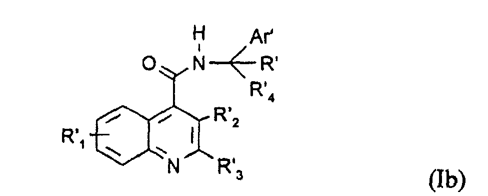 wherein Ar', R', R'₁, R'₂, R'₃ and R'₄ are as defined above, and thereafter carrying out one or more of the following optional steps:
(i) converting any one of Ar', R', R'₁, R'₂, R'₃ and R'₄ to Ar, R, R₁, R₂, R₃ or R₄ respectively as required, to obtain a compound of formula (I);
(ii) converting a compound of formula (I) into another compound of formula (I); and
(iii) preparing a salt of the compound of formula (I) and/or a solvate thereof.

Suitable groups convertible into other groups include protected forms of said groups.

Suitably Ar', R', R'₁, R'₂, R'₃ or R'₄ each represents Ar, R, R₁, R₂, R₃ or R₄ respectively or a protected form thereof.

It is favoured if the compound of formula (II) is present as an active derivative.

A suitable active derivative of a compound of formula (II) is a transient activated form of the compound of formula (II) or a derivative wherein the carboxy group of the compound of formula (II) has been replaced by a different group or atom, for example by an acyl halide, preferably a chloride, or an acylazide or a carboxylic acid anhydride.

Other suitable active derivatives include: a mixed anhydride formed between the carboxyl moiety of the compound of formula (II) and an alkyl chloroformate; an activated ester, such as a cyanomethyl ester, thiophenyl ester, p-nitrophenyl ester, p-nitrothiophenyl ester, 2,4,6-trichlorophenyl ester, pentachlorophenyl ester, pentafluorophenyl ester, N-hydroxy-phtalimido ester, N-hydroxypiperidine ester, N-hydroxysuccinimide ester, N-hydroxy benzotriazole ester; alternatively, the carboxy group of the compound of formula (II) may be activated using a carbodiimide or N,N'-carbonyldiimidazole.

The reaction between the compound of formula (II) or the active derivative thereof and the compound of formula (III) is carried out under the appropriate conventional conditions for the particular compounds chosen. Generally, when the compound of formula (II) is present as an active derivative the reaction is carried out using the same solvent and conditions as used to prepare the active derivative, preferably the active derivative is prepared *in situ* prior to forming the compound of formula (Ib) and thereafter the compound of formula (I) or a salt thereof and/or a solvate thereof is prepared.

For example, the reaction between an active derivative of the compound of formula (II) and the compound of formula (III) may be carried out:
(a) by first preparing an acid chloride and then coupling said chloride with the compound of formula (III) in the presence of an inorganic or organic base in a suitable aprotic solvent such as dimethylformamide (DMF) at a temperature in a range from -70 to 50°C (preferably in a range from -10 to 20°C); or
(b) by treating the compound of formula (II) with a compound of formula (III) in the presence of a suitable condensing agent, such as for example N,N'-carbonyl diimidazole (CDI) or a carbodiimide such as dicyclohexylcarbodiimide (DCC) or N-dimethylaminopropyl-N'-ethylcarbodiimide, preferably in the presence of N-hydroxybenzotriazole (HOBT) to maximise yields and avoid racemization processes (see *Synthesis,* 453, 1972), in an aprotic solvent, such as a mixture of acetonitrile (MeCN) and tetrahydrofuran (THF), for example a mixture in a volume ratio of from 1:9 to 7:3 (MeCN:THF), at any temperature providing a suitable rate of formation of the required product, such as a temperature in the range of from -70 to 50°C, preferably in a range of from -10 to 25°C, for example at 0°C.

A preferred reaction is set out in Scheme 1 shown below: 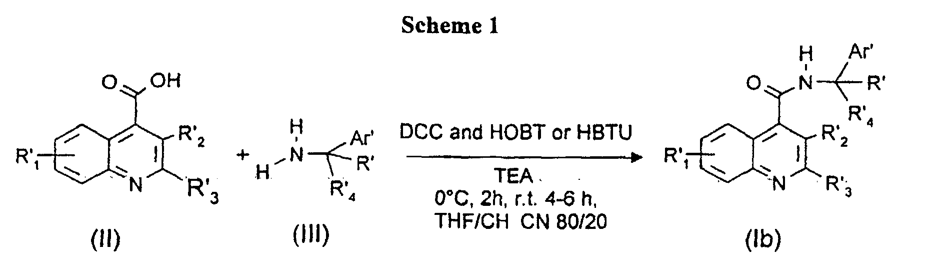 wherein Ar', R', R'₁, R'₂, R'₃ and R'₄ are as defined above.

It will be appreciated that a compound of formula (Ib) may be converted to a compound of formula (I), or one compound of formula (I) may be converted to another compound of formula (I) by interconversion of suitable substituents. Thus, certain compounds of formula (I) and (Ib) are useful intermediates in forming other compounds of the present invention.

Accordingly, in a further aspect the invention provides a process for preparing a compound of formula (I), or a salt thereof and/or a solvate thereof, which process comprises converting a compound of the above defined formula (Ib) wherein at least one of Ar', R', R'₁ R'₂, R'₃ or R'4 is not Ar, R, R₁, R₂, R₃ or R₄ respectively, thereby to provide a compound of formula (I): and thereafter, as required, carrying out one or more of the following optional steps:
(i) converting a compound of formula (I) into another compound of formula (I); and
(ii) preparing a salt of the compound of formula (I) and/or a solvate thereof.

Suitably, in the compound of formula (Ib) the variables Ar', R', R'₁ R'₂, R'₃ and R'₄ are Ar, R, R₁, R₂, R₃ or R₄ respectively or they are protected forms thereof.

The above mentioned conversions, protections and deprotections are carried out using the appropriate conventional reagents and conditions and are further discussed below.

A compound of formula (II) wherein n is an integer 1, is prepared by reacting a compound of formula (IV): 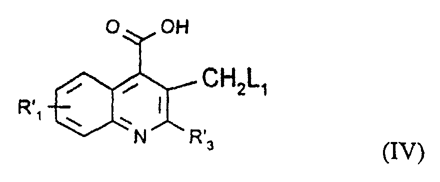 wherein R'₁ and R'₃ are as defined above and L₁ represents a halogen atom such as a bromine atom, with a compound of formula (V):

HNY'₁Y'₂ (V)

wherein Y'₁ and Y'₂ are respectively Y₁ and Y₂ as defined in relation to formula (I) or protected forms thereof.

Suitably, Y'₁ and Y'₂ are Y₁ and Y₂.

Suitably, reaction between the compounds of formulae (IV) and (V) is carried out under conventional amination conditions, for example when L₁ is a bromine atom then the reaction is conveniently carried out in an aprotic solvent, such as tetrahydrofuran or dimethylformamide at any temperature providing a suitable rate of formation of the required product, usually at ambient temperature; preferably the reaction is carried out in the presence of triethylamine (TEA).

A compound of formula (IV) is prepared by appropriate halogenation of a compound of formula (VI): 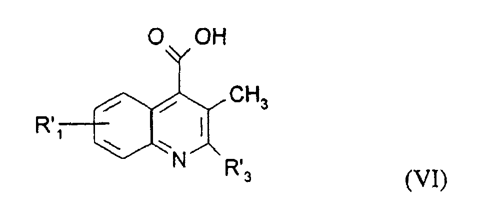 wherein R'₁ and R'₃ are as defined above in relation to formula (II).

Suitable halogenation reagents are conventional reagents depending upon the nature of the halogen atom required, for example when L₁ is bromine a preferred halogenation reagent is N-bromosuccinimide (NBS).

The halogenation of the compound of formula (VI) is carried out under conventional conditions, for example bromination is carried out by treatment with NBS in an inert solvent, such as 1,2-dichloroethane, at any temperature providing a suitable rate of formation of the required product, suitably at an elevated temperature such as a temperature in the range of 60°C to 100°C, for example 80°C; preferably the reaction is carried out in the presence of a catalytic amount on benzoyl peroxide.

A compound of formula (II) wherein R₂ represents -(CH₂)₂₋₉-NY₁Y₂, is conveniently prepared by reacting a compound of formula (VII): 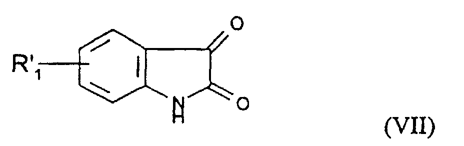 wherein R'₁ is as defined in relation to formula (II), with a compound of formula (VIII):

R₃'―CO―CH₂―(CH₂)p―NY₁Y₂ (VIII)

wherein R'₃ is as defined in relation to formula (II), Y₁ and Y₂ are as defined in relation to formula (I) and p is an integer in the range of 2 to 9.

The reaction between the compounds of formula (VII) and (VIII) is conveniently carried out using Pfitzinger reaction conditions (see for example J. Prakt., Chem. 33, 100 (1886), J.. Prakt. Chem. 38, 582 (1888), J. Chem. Soc. 106 (1948) and Chem. Rev. 35, 152 (1944)), for example in an alkanolic solvent such as ethanol, at any temperature providing a suitable rate of formation of the required product, but generally at an elevated temperature, such as the reflux temperature of the solvent, and preferably in the presence of a base such as potassium hydroxide or potassium tert-butoxide.

A compound of formula (VIII) is prepared from a compound of formula (IX):

R₃'―CO―CH₂―(CH₂)ₚ―OH (IX)

wherein R'₃ is as defined in relation to formula (II) and p is as defined in relation to formula (VIII), by first halogenating, preferably brominating, or mesylating the compound of formula (IX) and thereafter reacting the halogenation or mesylation product so formed with a compound of the above defined formula (V).

The halogenation of the compound of formula (IX) is suitably carried out using a conventional halogenation reagent. Mesylation is conveniently carried out using mesyl chloride in an inert solvent such as methylene dichloride, at a temperature below room temperature, such as 0°C, preferably in the presence of triethylamine.

The reaction between the halogenation or mesylation product of the compound of formula (IX) and the compound of formula (V) is carried out under analogous conditions to those described for the reaction between the compounds of formulae (IV) and (V).

A compound of formula (IX) may be prepared by reacting a compound of formula (X): 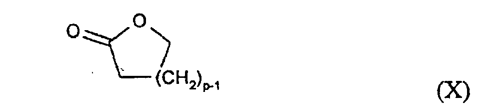 wherein p is as defined in relation to formula (VIII), with a lithium salt of formula (XI):

R'₃ Li (XI)

wherein R'₃ is as defined in relation to formula (II).

The reaction between the compounds of formulae (X) and (XI) can be carried out in an aprotic solvent, such as diethyl-ether at any temperature providing a suitable rate of formation of the required product, usually at a low temperature such as in the range of -10°C to -30°C, for example -20°C.

The compounds of formula (III) are known commercially available compounds or they can be prepared from known compounds by known methods, or methods analogous to those used to prepare known compounds, for example the methods described in Liebigs Ann. der Chemie, 523, 199, 1936.

The compounds of formula (V) are known, commercially available compounds or they can be prepared using methods analogous to those used to prepare known compounds; for example the methods described in the Chemistry of the Amino Group, Patais (Ed.), Interscience, New York 1968 or Advanced Organic Chemistry, March J, John Wiley & Sons, New York, 1992.

The compounds of formula (VII) are known compounds or they are prepared according to methods used to prepare known compounds for example those disclosed in J. Org. Chem. 21, 171 (1955); J. Org. Chem. 21, 169 (1955).

The compounds of formula (X) and (XI) are known compounds or they are prepared according to methods used to prepare known compounds for example those disclosed by Krow G. R. in Organic Reactions, Vol 43, page 251, John Wiley & Sons Inc.1994 (for the compounds of formula (X)) and Organometallics in Synthesis, Schlosser M.(Ed), John Wiley & Sons Inc.1994 (for the compounds of formula (XI))

As hereinbefore mentioned, the compounds of formula (I) may exist in more than one stereoisomeric form - and the process of the invention may produce racemates as well as enantiomerically pure forms. Accordingly, a pure enantiomer of a compound of formula (I) is obtained by reacting a compound of the above defined formula (II) with an appropriate enantiomerically pure primary amine of formula (IIIa) or (IIIc): 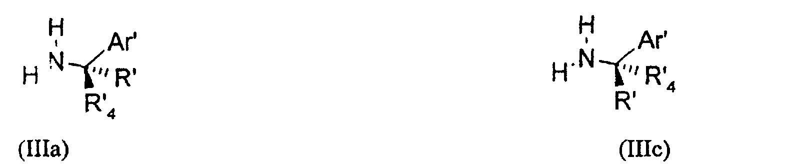 wherein R', R'₄and Ar' are as defined above, to obtain a compound of formula (I'a) or (I'c): 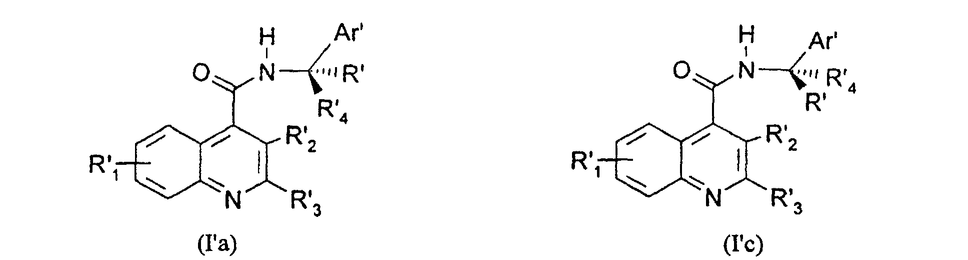 wherein Ar', R', R'₁, R'₂, R'₃ and R'₄ are as defined above.

Compounds of formula (I'a) or (I'c) may subsequently be converted to compounds of formula (Ia) or (Ic) by the methods of conversion mentioned before: 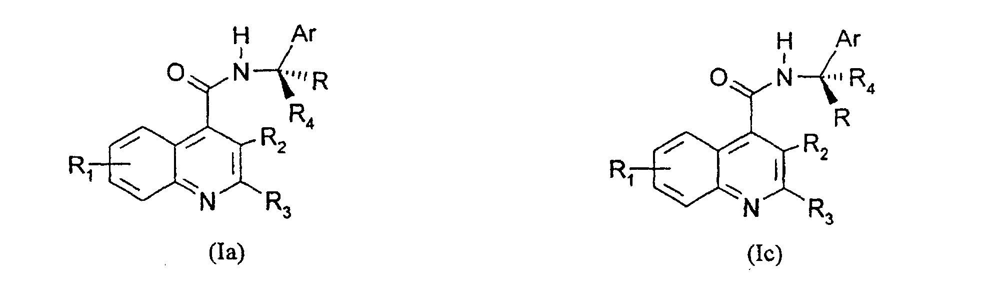 wherein Ar, R, R₁ R₂, R₃ and R₄ are as defined above.

Suitably, in the above mentioned compounds of formulae (Ia), (Ic), (I'a), (I'c), (IIl'a) and (III'c) R₄ represents hydrogen. An alternative method for separating optical isomers, for example for those compounds of formula (I) wherein R₄ is different from hydrogen, is to use conventional, fractional separation methods in particular fractional crystallization methods. Thus, a pure enantiomer of a compound of formula (I) is obtained by fractional crystallisation of a diastereomeric salt formed by reaction of the racemic compound of formula (I) with an optically active strong acid resolving agent, such as camphosulphonic acid, in an appropriate alcoholic solvent, such as ethanol or methanol, or in a ketonic solvent, such as acetone. The salt formation process should be conducted at a temperature between 20°C and 80°C, preferably at 50°C.

In the case in which other basic functionalities, such as primary, secondary or tertiary amine, are present in the molecule, a wider range of optically active acid resolving agents become available, including tartaric acid, O,O'-di-p-toluoyitartaric acid and mandelic acid.

A suitable conversion of one compound of formula (I) into a further compound of formula (I) involves converting one group R₂ into another group R₂ by for example:
(i) converting a ketal into a ketone, by such as mild acidic hydrolysis, using for example dilute hydrochloric acid;
(ii) reducing a ketone to a hydroxyl group by use of a borohydride reducing agent;
(iii) converting a carboxylic ester group into a carboxyl group using basic hydrolysis; and/or
(iv) reducing a carboxylic methyl ester group to a hydroxymethyl group, by use of a borohydride reducing agent.

As indicated above, where necessary, the conversion of any group Ar', R', R'₁ R'₂, R'₃ and R'₄ into Ar, R, R₁, R₂, R₃ or R₄ which as stated above are usually protected forms of Ar, R, R₁, R₂, R₃ or R₄ may be carried out using appropriate conventional conditions such as the appropriate deprotection procedure.

It will be appreciated that in any of the above mentioned reactions any reactive group in the substrate molecule may be protected and deprotected according to conventional chemical practice, for example as described by Greene, T.W. and Wuts, P.G.M. Protective Groups in Organic Synthesis, John Wiley & Sons Inc. New York, 1991 (Second Edt.) or in Kocienski, P.J. Protecting groups. George Thieme Verlag, New York, 1994.

Suitable protecting groups in any of the above mentioned reactions are those used conventionally in the art. Thus, for example suitable hydroxyl protecting groups include benzyl or trialkylsilyl groups.

The methods of formation and removal of such protecting groups are those conventional methods appropriate to the molecule being protected. Thus for example a benzyloxy group may be prepared by treatment of the appropriate compound with a benzyl halide, such as benzyl bromide, and thereafter, if required, the benzyl group may be conveniently removed using catalytic hydrogenation or a mild ether cleavage reagent such as trimethylsilyl iodide or boron tribromide.

As indicated above, the compounds of formula (I) have useful pharmaceutical properties.

Accordingly the present invention also provides a compound of formula (I), or a pharmaceutically acceptable salt or solvate thereof, for use as an active therapeutic substance.

In particular, the present invention also provides a compound of formula (I), or a pharmaceutically acceptable salt or solvate thereof, for the treatment or prophylaxis of the Primary and Secondary Conditions.

The present invention further provides a pharmaceutical composition comprising a compound of formula (I), or a pharmaceutically acceptable salt or solvate thereof, and a pharmaceutically acceptable carrier.

The present invention also provides the use of a compound of formula (I), or a pharmaceutically acceptable salt or solvate thereof, in the manufacture of a medicament for the treatment of the Primary and Secondary Conditions.

As mentioned abvove the Primary conditions include respiratory diseases, such as chronic obstructive pulmonary disease (COPD), asthma, airway hyperreactivity, cough; inflammatory diseases such as inflammatory bowel disease, psoriasis, fibrositis, osteoarthritis, rheumatoid arthritis and inflammatory pain; neurogenic inflammation or peripheral neuropathy, allergies such as eczema and rhinitis; ophthalmic diseases such as ocular inflammation, conjunctivitis, vernal conjuctivitis and the like; cutaneous diseases, skin disorders and itch, such as cutaneous wheal and flare, contact dermatitis, atopic dermatitis, urticaria and other eczematoid dermatitis; adverse immunological reactions such as rejection of transplanted tissues and disorders related to immune enhancement or suppression such as systhemic lupus erythematosis; gastrointestinal (GI) disorders and diseases of the GI tract such as disorders associated with the neuronal control of viscera such as ulcerative colitis, Crohn's disease and urinary incontinence; renal disorders and disorders of the bladder function, especially chronic obstructive pulmonary disease (COPD), urinary incontinence; renal disorders and disorders of the bladder function.

As mentioned abvove,the Secondary conditions disorders of the central nervous system such as anxiety, depression, psychosis and schizophrenia; neurodegenerative disorders such as AIDS related dementia, senile dementia of the Alzheimer type, Alzheimer's disease, Down's syndrome, Huntington's disease, Parkinson's disease, movement disorders and convulsive disorders (for example epilepsy); demyelinating diseases such as multiple sclerosis and amyotrophic lateral sclerosis and other neuropathological disorders such as diabetic neuropathy, AIDS related neuropathy, chemotherapy-induced neuropathy and neuralgia; addiction disorders such as alcoholism; stress related somatic disorders; reflex sympathetic dystrophy such as shoulder/hand syndrome; dysthymic disorders; eating disorders (such as food intake disease); fibrosing and collagen diseases such as scleroderma and eosinophilic fascioliasis; disorders of the blood flow caused by vasodilation and vasospastic diseases such as angina, migraine and Reynaud's disease and pain or nociception, for example, that is attributable to or associated with any of the foregoing conditions especially the transmission of pain in migraine.

Such a medicament, and a composition of this invention, may be prepared by admixture of a compound of the invention with an appropriate carrier. It may contain a diluent, binder, filler, disintegrant, flavouring agent, colouring agent, lubricant or preservative in conventional manner.

These conventional excipients may be employed for example as in the preparation of compositions of known agents for treating the conditions.

Preferably, a pharmaceutical composition of the invention is in unit dosage form and in a form adapted for use in the medical or veterinarial fields. For example, such preparations may be in a pack form accompanied by written or printed instructions for use as an agent in the treatment of the conditions.

The suitable dosage range for the compounds of the invention depends on the compound to be employed and on the condition of the patient. It will also depend, inter alia, upon the relation of potency to absorbability and the frequency and route of administration.

The compound or composition of the invention may be formulated for administration by any route, and is preferably in unit dosage form or in a form that a human patient may administer to himself in a single dosage. Advantageously, the composition is suitable for oral, rectal, topical, parenteral, intravenous or intramuscular administration. Preparations may be designed to give slow release of the active ingredient.

Compositions may, for example, be in the form of tablets, capsules, sachets, vials, powders, granules, lozenges, reconstitutable powders, or liquid preparations, for example solutions or suspensions, or suppositories.

The compositions, for example those suitable for oral administration, may contain conventional excipients such as binding agents, for example syrup, acacia, gelatin, sorbitol, tragacanth, or polyvinylpyrrolidone; fillers, for example lactose, sugar, maize-starch, calcium phosphate. sorbitol or glycine; tabletting lubricants, for example magnesium stearate; disintegrants, for example starch, polyvinyl-pyrrolidone, sodium starch glycollate or microcrystalline cellulose; or pharmaceutically acceptable setting agents such as sodium lauryl sulphate.

Solid compositions may be obtained by conventional methods of blending, filling, tabletting or the like. Repeated blending operations may be used to distribute the active agent throughout those compositions employing large quantities of fillers. When the composition is in the form of a tablet, powder, or lozenge, any carrier suitable for formulating solid pharmaceutical compositions may be used, examples being magnesium stearate, starch, glucose, lactose, sucrose, rice flour and chalk. Tablets may be coated according to methods well known in normal pharmaceutical practice, in particular with an enteric coating. The composition may also be in the form of an ingestible capsule, for example of gelatin containing the compound, if desired with a carrier or other excipients.

Compositions for oral administration as liquids may be in the form of, for example, emulsions, syrups, or elixirs, or may be presented as a dry product for reconstitution with water or other suitable vehicle before use. Such liquid compositions may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; aqueous or non-aqueous vehicles, which include edible oils, for example almond oil, fractionated coconut oil, oily esters, for example esters of glycerine, or propylene glycol, or ethyl alcohol, glycerine, water or normal saline; preservatives, for example methyl or propyl p-hydroxybenzoate or sorbic acid; and if desired conventional flavouring or colouring agents.

The compounds of this invention may also be administered by a non-oral route. In accordance with routine pharmaceutical procedure, the compositions may be formulated, for example for rectal administration as a suppository. They may also be formulated for presentation in an injectable form in an aqueous or non-aqueous solution, suspension or emulsion in a pharmaceutically acceptable liquid, e.g. sterile pyrogen-free water or a parenterally acceptable oil or a mixture of liquids. The liquid may contain bacteriostatic agents, anti-oxidants or other preservatives, buffers or solutes to render the solution isotonic with the blood, thickening agents, suspending agents or other pharmaceutically acceptable additives. Such forms will be presented in unit dose form such as ampoules or disposable injection devices or in multi- dose forms such as a bottle from which the appropriate dose may be withdrawn or a solid form or concentrate which can be used to prepare an injectable formulation.

The compounds of this invention may also be administered by inhalation, via the nasal or oral routes. Such administration can be carried out with a spray formulation comprising a compound of the invention and a suitable carrier, optionally suspended in, for example, a hydrocarbon propellant.

Preferred spray formulations comprise micronised compound particles in combination with a surfactant, solvent or a dispersing agent to prevent the sedimentation of suspended particles. Preferably, the compound particle size is from about 2 to 10 microns.

A further mode of administration of the compounds of the invention comprises transdermal delivery utilising a skin-patch formulation. A preferred formulation comprises a compound of the invention dispersed in a pressure sensitive adhesive which adheres to the skin, thereby permitting the compound to diffuse from the adhesive through the skin for delivery to the patient. For a constant rate of percutaneous absorption, pressure sensitive adhesives known in the art such as natural rubber or silicone can be used.

As mentioned above, the effective dose of compound depends on the particular compound employed, the condition of the patient and on the frequency and route of administration. A unit dose will generally contain from 20 to 1000 mg and preferably will contain from 30 to 500 mg, in particular 50, 100, 150, 200, 250, 300, 350, 400, 450, or 500 mg. The composition may be administered once or more times a day for example 2, 3 or 4 times daily, and the total daily dose for a 70 kg adult will normally be in the range 100 to 3000 mg. Alternatively the unit dose will contain from 2 to 20 mg of active ingredient and be administered in multiples, if desired, to give the preceding daily dose.

No unacceptable toxicological effects are expected with compounds of the invention when administered in accordance with the invention.

The present invention also provides a method for the treatment and/or prophylaxis of the Primary and Secondary Conditions in mammals, particularly humans, which comprises administering to the mammal in need of such treatment and/or prophylaxis an effective, non-toxic pharmaceutically acceptable amount of a compound of formula (I) or a pharmaceutically acceptable salt or solvate thereof.

The activity of the compounds of the present invention, as NK₃ ligands, is determined by their ability to inhibit the binding of the radiolabelled NK₃ ligands, [¹²⁵I]-[Me-Phe⁷]-NKB or [³H]-Senktide, to guinea-pig and human NK₃ receptors (Renzetti et al, **1991**, *Neuropeptide, 18,* 104-114; Buell et al, **1992,** *FEBS, 299(1)*, 90-95; Chung et al, **1994**, *Biochem. Biophys. Res. Commun., 198(3)*, 967-972).

The binding assays utilized allow the determination of the concentration of the individual compound required to reduce by 50% the [¹²⁵I]-[Me-Phe⁷]-NKB and [³H]-Senktide specific binding to NK₃ receptor in equilibrium conditions (IC50).

Binding assays provide for each compound tested a mean IC₅₀ value of 2-5 separate experiments performed in duplicate or triplicate. The most potent compounds of the present invention show IC₅₀ values in the range 0.1-1000 nM. The NK₃-antagonist activity of the compounds of the present invention is determined by their ability to inhibit senktide-induced contraction of the guinea-pig ileum (Maggi et al, **1990**, *Br. J. Pharmacol., 101*, 996-1000) and rabbit isolated iris sphincter muscle (Hall et al., **1991,** *Eur. J. Pharmacol., 199,* 9-14) and human NK₃ receptors-mediated Ca⁺⁺ mobilization (Mochizuki et al, **1994,** *J. Biol. Chem., 269,* 9651-9658). Guinea-pig and rabbit *in-vitro* functional assays provide for each compound tested a mean K_{B} value of 3-8 separate experiments, where K_{B} is the concentration of the individual compound required to produce a 2-fold rightward shift in the concentration-response curve of senktide. Human receptor functional assay allows the determination of the concentration of the individual compound required to reduce by 50% (IC₅₀ values) the Ca⁺⁺ mobilization induced by the agonist NKB. In this assay, the compounds of the present invention behave as antagonists.

The activity of the compounds of the present invention, as NK-2 ligands, is determined by their ability to inhibit the binding of the radiolabelled NK-2 ligands, [¹²⁵I]-NKA or [³H]-NKA, to human NK-2 receptors (Aharony et al, 1992, *Neuropeptide*, 23, 121-130).

The binding assays utilized allow the determination of the concentration of the individual compound required to reduce by 50% the [¹²⁵I]-NKA and [³H]-NKA specific binding to NK2 receptor in equilibrium conditions (IC₅₀).

Binding assays provide for each compound tested a mean IC₅₀ value of 2-5 separate experiments performed in duplicate or triplicate. The most potent compounds of the present invention show IC₅₀ values in the range 0.5-1000 nM, such as 1-1000 nM. The NK-2-antagonist activity of the compounds of the present invention is determined by their ability to inhibit human NK-2 receptor-mediated Ca⁺⁺ mobilization (Mochizuki et al, **1994,** *J. Biol. Chem., 269*, 9651-9658). Human receptor functional assay allows the determination of the concentration of the individual compound required to reduce by 50% (IC₅₀ values) the Ca⁺⁺ mobilization induced by the agonist NKA. In this assay, the compounds of the present invention behave as antagonists.

The therapeutic potential of the compounds of the present invention in treating the conditions can be assessed using rodent disease models.

As stated above, the compounds of formula (I) are also considered to be useful as diagnostic tool. Accordingly, the invention includes a compound of formula (I) for use as diagnostic tools for assessing the degree to which neurokinin-3 and neurokinin-2 receptor activity (normal, overactivity or underactivity) is implicated in a patient's symptoms. Such use comprises the use of a compound of formula (I) as an antagonist of said activity, for example including but not restricted to tachykinin agonist-induced inositol phosphate turnover or electrophysiological activation, of a cell sample obtained from a patient. Comparison of such activity in the presence or absence of a compound of formula (I), will disclose the degree of NK-3 and NK-2 receptor involvement in the mediation of agonist effects in that tissue.

The following Descriptions illustrate the preparation of the intermediates, whereas the following Reference Examples illustrate the preparation of the compounds of the invention.

### Descriptions and Examples

### DESCRIPTION 1: 3-[(S)-2-(Methoxycarbonyl)pyrrolidin-1-yl]-2-phenylquinoline-4-carboxylic acid hydrochloride

10 g (37.98 mmol) of 3-methyl-2-phenylquinoline-4-carboxylic acid (CAS [43071-45-0]) were suspended in 1000 ml of 1,2-dichloroethane; 13.67 g (76.80 mmol) of N-bromosuccinimide and 1.0 g (4.13 mmol) of dibenzoyl peroxide were added and the suspension was refluxed for 24 hours.

After cooling, the reaction mixture was evaporated *in-vacuo* and dissolved in 150 ml of anhydrous THF. Then 19.63 g (193.99 mmol) of TEA and 19.68 g (118.89 mmol) of L-proline methyl ester hydrochloride were added, the mixture was stirred at room temperature for 6 hours and evaporated *in-vacuo* to dryness. The crude oil was dissolved in a K₂CO₃ saturated solution, and evaporated *in vacuo* to dryness. The solid residue mixture was washed with Et₂O, acidified with HCl 6N and evaporated *in-vacuo* to dryness. The crude solid was triturated in MeOH, the solution was filtered off, concentrated *in vacuo*, and resulting oil was purified by gradient column chromatography on 70-230 mesh silica gel using a mixture of CH₂Cl₂/MeOH 90:10 containing 1 % NH₄OH (28%) as starting eluent and a mixture of CH₂Cl₂/MeOH 70:30 containing 1 % NH₄OH (28%) as final eluent. The obtained product was dissolved in Et₂O and acidified with HCl/Et₂O and the precipitate so formed was recovered by suction, affording 0.56 g of the title compound as brownish solid.
C₂₃H₂₂N₂O₄·HCl
MW 426.90
MP > 90°C (dec)
IR (neat) 3424, 2968, 1712, 1632 cm⁻¹.

### REFERENCE EXAMPLE 1 : (S)-N-(α-Ethylbenzyl)-3-[(S)-2-(methoxycarbonyl)pyrrolidin-1-yl]-2-phenylquinoline-4-carboxamide hydrochloride

1.0 g (2.56 mmol) of 3-[(S)-2-(Methoxycarbonyl)pyrrolidin-1-yl]-2-phenylquinoline-4-carboxylic acid were dissolved, under nitrogen atmosphere, in 30 ml of a 80:20 mixture of THF/CH₃CN. The solution was cooled at 0 °C and 0.38 g (2.81 mmol) of HOBT, 0.36 g (3.58 mmol) of TEA and 0.42 g (mmol 3.06) of (S)-α-ethylbenzylamine were added. The solution was stirred 5 minutes and 0.58 g (2.81 mmol) of DCC, dissolved in 18 ml of CH₂Cl₂, were added dropwise. The mixture was stirred 2 hours at 0 °C, then the temperature was allowed to raise to room temperature and the reaction was maintained under stirring for 3 hours. The precipitated dicyclohexylurea was filtered off and the filtrate was evaporated *in vacuo* to dryness. The residue was dissolved in CH₂Cl₂ and washed with IN NaOH. The organic layer was dried over Na₂SO₄ and evaporated *in vacuo* to dryness. The crude product was purified by gradient column chromatography on 70-230 mesh silica gel using a mixture of hexane/EtOAc 90:10 as starting eluent and a mixture of hexane/EtOAc 70/30 containing 1% NH₄OH (28%) as final eluent. The product obtained was dissolved in Et₂O and acidified with HCl/Et₂O; the precipitate so formed was recovered by suction filtration affording 0.29 g of the title compound.
C₃₂H₃₃N₃O₃·HCl
MW = 544.10
MP > 127°C (dec)
[α]_{D}²⁰ = - 38.6 (c = 0.06; MeOH)
IR (KBr) 3434, 2932, 1746, 1664, 1552, 764, 704 cm⁻¹.
MS: A) ESI POS; TSQ 700; solvent: methanol/ spray 4.5 kV / skimmer: 60 V/ capillary 220 C; B) ESI DAU+508 (Collision gas: Argon). *m*/*z*: A) 508 (MH+); B) 508; 373; 261; 244; 220; 119.
- 300 MHz 1H-NMR (DMSO-d₆) (353K):: δ 8.75 (d br, 1H); 8.00 (d, 1H); 7.74 (m, 2H); 7.58-7.42 (m, 8H); 7.38 (dd, 2H); 7.29 (dd, 1H); 5.11 (dt, 1H); 3.89 (s, 2H); 3.29 (s, 3H); 2.99 (dd, 1H); 2.46 (m, 1H); 2.26 (m, 1H); 2.04-1.80 (m, 2H); 1.78-1.40 (m, 4H); 0.97 (t, 3H).

### REFERENCE EXAMPLE 2: (S)-N-(α-Ethylbenzyl)-3-[(S)-2-(hydroxymethyl)pyrrolidin-1-yl]-2-phenylquinoline-4-carboxamide

1.6 g (3.2 mmol) of (S)-N-(α-Ethylbenzyl)-3-[(S)-2-(methoxycarbonyl)pyrrolidin-1-yl]-2-phenylquinoline-4-carboxamide (compound of Example 1) were dissolved, under nitrogen atmosphere, in 40 ml of t-BuOH, 0.6 g (15.86 mmol) of NaBH₄ were added and the solution was heated to 80°C. Than 5 ml of MeOH were added and the solution refluxed 6 hours. After cooling, the reaction was quenched with 10 ml of water and 10 ml of 6N NaOH. The solution was evaporated *in vacuo* to dryness and the residue was dissolved in EtOAc. 1.5 g of Silica gel were added, the suspension was stirred 1 hour, filtered off and evaporated *in vacuo* to dryness, to affords 0.4 g of the title compound.
C₃₁H₃₃N₃O₂
MW = 479.63
MP 85-89°C
[α]_{D}²⁰ = - 83.5 (c = 0.12; MeOH)
IR: (KBr) 3420, 3251, 1638 cm⁻¹.
MS: A) ESI POS; TSQ 700; solvent: methanol/ spray 4.5 kV / skimmer: 60 V/ capillary 220 C; B) ESI DAU+480 (Collision gas: Argon). *m*/*z*: A) 480 (MH+); B) 480; 345; 273; 261; 218; 119.
- 300 MHz ¹H-NMR (DMSO-d₆) (353K):: δ 8.89 (d, 1H); 7.99 (d, 1H); 7.72 (ddd, 1H); 7.66 (d, 1H); *7.55-7.42* (m, 8H); 7.39 (dd, 2H); 7.30 (dd, 1H); 5.09 (dt, 1H); 4.02 (d, 1H); 3.62 (d, 1H); 3.38 (m, 1H); 2.91 (m, 1H); 2.79 (m, 1H); 2.28 (m, 1H); 2.02 (m, 1H); 2.00-1.81 (m, 2H); 1.68 (m, 1H); 1.45-1.30 (m, 3H); 1.00 (t, 3H).

### REFERENCE EXAMPLE 3: (S)-N-(α-Ethylbenzyl)-3-[(S)-2-carboxypyrrolidin-1-yl]-2-phenylquinoline-4-carboxamide

The Silica gel filtered in the last step of Example 2 was suspended in MeOH and stirred for 2 hours. The mixture was filtered off and evaporated *in vacuo* to dryness, to afford 0.24 g of the title compound.
C₃₁H₃₁N₃O₃
MW = 493.61
MP 188-192°C [α]_{D}²⁰ = -29.7 (c = 0.5; MeOH)
IR: (mull oil) 3600-3200, 1640, 1600-1500, 764, 702 cm⁻¹.
MS: A) ESI POS; TSQ 700; solvent: methanol/ spray 4.5 kV / skimmer: 60 V/ capillary 220 C; B) ESI DAU+494, 308(Collision gas: Argon); C) ESI NEG; TSQ 700; solvent: methanol/ spray -4.5 kV / skimmer: 60 V/ capillary 220 C; D) ESI DAU-492, 308(Collision gas: Argon). *m*/*z*: A) 494 (MH+); 516 (MNa+); B) 516; 470; 444, 403; 353; C) 492 (M-H-); D) 492; 448; 287; 218; 204.
- 300 MHz ¹H-NMR (DMSO-d₆) (333K):: δ 11.85 (s br, 1H); 7.93 (d, 1H); 7.75-7.51 (m, 6H); 7.42-7.35 (m, 7H); 5.10 (dt, 1H); 3.85 (m, 1H); 3.75 (d, 1H); 3.60 (d, 1H); 2.80 (dd, 1H); 2.41 (ddd, 1H); 2.09-1.96 (m, 2H); 1.90-1.80 (m, 1H); 1.70-1.60 (m, 2H); 1.45-1.32 (m, 1H); 0.95 (t, 3H).

Following the same procedure described in Example 1 and starting from the appropriate aminoacids of formula II (prepared as described in Description 1), and secondary amines of formula PhCH(NH₂)R, compounds of Examples 4, 6-11, 15, 16, 19, 20, 26, 31-33, 36-38, 41 and 43 were prepared.
Compound of Example **5** was prepared as described in the procedure of Example **2.**

### DESCRIPTION 2: 4-Phenyl-4-oxobutanol

11.2 g (130.01 mmol) of γ-Butyrolactone were dissolved, under nitrogen atmosphere, in 50 ml of dry THF. The solution was cooled to -78°C and 21.7 ml (43.4 mmol) of 2 M phenyl lithium in diethyl ether were added dropwise keeping the temperature between -60 and -70°C. The temperature was allowed to rise to room temperature and the solution was stirred 1 hour. The reaction was quenched with 30 ml NH₄Cl 10%, diluted with water, extracted with Et₂O and evaporated *in vacuo* to dryness. The crude product was dissolved in 100 ml of EtOH, 10 g of KOH pellets were added and the mixture was maintained at room temperature overnight. The solvent was evaporated *in vacuo* to dryness and the residue, dissolved in water, was extracted with Et₂O. The organic layer was dried over Na₂SO₄, evaporated *in vacuo* to dryness and purified by flash column chromatography on 230-400 mesh silica gel, utilising a mixture of hexane/EtOAc 7:3 as eluent, to afford 8 g of the title compound.
C₁₀H₁₂O₂
MW = 164.20
IR: (neat) 3375, 3060, 2952, 1674, 1598 cm⁻¹.

### DESCRIPTION 3: 4-(4-phenyl-4-oxo)butyl-1-phenylpiperazine

5 g (30.40 mmol) of 4-Phenyl-4-oxobutanol and 4 g (39.51 mmol) of TEA were diluted, under nitrogen atmosphere, in 80 ml of dry CH₂Cl₂. The solution was cooled to 0°C and 4.12 g (35.93 mmol) of methanesulphonyl chloride were added dropwise keeping the temperature between 0 and 5°C. The reaction was allowed to rise to room temperature and stirred 1 hour. The solution was then washed with 40 ml of cool water and the organic layer dried over Na₂SO₄ The crude oil was dissolved in ml 25 of DMF, 9.82 g (60.80 mmol) of phenylpiperazine were added and the stirring was maintained for 4 hours at room temperature. The reaction mixture was evaporated *in vacuo*, the residue treated with EtOAc and filtered off. The organic phase was washed with H₂O, evaporated *in vacuo* to dryness and purified by flash column chromatography on 230-400 mesh silica gel eluting with (i-Pr)₂O/Et₂O 1: I to afford 2.1 g of the title compound.
C₂₀H₂₄N₂O
MW = 308.43
IR: (KBr) 3456, 3062, 3020, 2880, 1684, 1600, 1502, 754, 736, 688 cm⁻¹.

### DESCRIPTION 4: 2-Phenyl-3-[2-(4-phenylpiperazin-1-yl)ethyl]-4-quinolinecarboxylic acid

1.15 g (7.80 mmol) of Isatin were suspended in 20 ml of absolute EtOH and 1.4 g (21.45 mmol) of 85% KOH were added. After stirring 30 minutes at room temperature 2 g (6.50 mmol) of 4-(4-phenyl-4-oxo)butyl-1-phenylpiperazine were added and the mixture refluxed 2 days. After cooling to room temperature, the solution was acidified with 6 N HCl to pH = 7 and extracted with EtOAc. The organic layer was dried over Na₂SO₄ and evaporated *in vacuo* to dryness. The residue was purified by flash column chromatography on 230-400 mesh silica gel eluting with AcOEt/MeOH 80:20 containing 2% NH₄OH (28%). The crude yellow solid was triturated with warm EtOAc to afford 1.24 g of the title compound as a white powder.
C₂₈H₂₇N₃O₂
MW = 437.55
IR: (KBr) 3432, 3060, 2600-2200, 1736, 1604, 1498, 760, 700 cm⁻¹.

Following the same procedure described in Example 1 and starting from the appropriate aminoacids of formula **II** described in Description 4, compounds of Examples **12-14, 21-25, 27, 29, 30, 35, 39, 40** and **42** were prepared.

### REFERENCE EXAMPLE 17: (S)-N-(α-Ethylbenzyl)-3-[(4-oxopiperidin-1-yl)methyl]-2-phenylquinoline-4-carboxamide

0.1 g (0.192 mmol) of (S)-N-(α-ethylbenzyl)-3-[(1,4-dioxa-8-azaspiro[4.5]decan-8-yl)methyl]-2-phenylquinoline-4-carboxamide (compound of Example 9) were dissolved in 20 ml of 6 N HCl and stirred at room temperature for 24 hours. The solution was basified with 6 N NaOH and extracted with EtOAc. The organic layer was dried over Na₂SO₄ and evaporated *in vacuo* to dryness to afford 0.040 g of the title compound.
C₃₁H₃₁N₃O₂
MW = 477.61
MP > 95°C
[α]_{D}²⁰ = -44.0 (c = 0.36; MeOH)
IR: (KBr) 3299, 3059, 2964, 2933, 1714, 1634, 1533, 1492, 763, 701 cm⁻¹.
MS: A) ESI POS; TSQ 700; solvent: methanol/ spray 4.5 kV / skimmer: 60 V/ capillary 220 C; B) ESI DAU+478(Collision gas: Argon); *m*/*z:* A) 478 (MH+); 510 (MNa+); B) 343; 314; 273; 261; 244; 220.
- 300 MHz ¹H-NMR (DMSO-d₆):: 8.87 (d br, 1H); 8.02 (d, 1H); 7.79-7.72 (m, 2H); 7.59 (dd, 2H); 7.52-7.42 (m, 5H); 7.36 (dd, 2H); 7.28 (dd, 1H); 5.11 (dt, 1H); 3.65 (s, 2H); 2.39-2.22 (m, 4H); 1.99-1.92 (m, 4H); 1.89 (dq, 2H); 0.98 (t, 3H).

### REFERENCE EXAMPLE 18: (S)-N-(α-Ethylbenzyl)-3-[(4-hydroxypiperidin-1-yl)methyl]-2-phenylquinoline-4-carboxamide

0.38 g (0.79 mmol) of (S)-N-(α-ethylbenzyl)-3-[(4-oxopiperidin-1-yl)methyl]-2-phenylquinoline-4-carboxamide (compound of Example 17) were dissolved in 15 ml of MeOH. The solution was cooled to 5°C and 0.095 g of NaBH₄ (2.5 mmol) were added portionwise. The reaction was stirred 3 hours at 5°C and quenched with 6 N HCl. The solvent was evaporated *in vacuo* and the crude oil suspended in 6 N NaOH and extracted with CH₂Cl₂. The organic layer was dried over Na₂SO₄ and evaporated *in vacuo* to dryness. The crude oil was purified by column chromatography on 70-230 mesh silica gel, eluting with a mixture of EtOAc/hexane 60:40 containing 1% NH₄OH (28%), to afford 0.32 g of title compound.
C₃₁H₃₃N₃O₂
MW = 479.63
MP = 203-207°C
[α]_{D}²⁰ = - 48.7 (c = 0.4; MeOH)
IR: (KBr) 3400, 3294, 1640, 1536, 1492, 1132, 1062, 764, 700 cm⁻¹.
MS: A) ESI POS; TSQ 700; solvent: methanol/ spray 4.5 kV / skimmer: 60 V/ capillary 220 C; B) CID OFFset = -49V; *m*/*z:* A) 480 (MH+); B) 480; 345; 261; 244; 216.
- 300 MHz ¹H-NMR (DMSO-d₆): (373 K):: 8.69 (d br, 1H); 8.00 (d, 1H); 7.71 (m, 2H); 7.58-7.27 (m, 11H); 5.11 (dq, 1H); 3.79 (d, 1H); 3.50 (s, 2H); 3.32-3.22 (m, 1H); 2.47 (m, 2H); 2.30 (m,2H); 2.01-1.82 (m, 2H); 1.80-1.70 (m, 2H); 1.43 (m, 2H); 0.99 (t,

### REFERENCE EXAMPLE 28: (S)-N-(α-Ethylbenzyl)-3-[(piperazin-1-yl)methyl]-2-phenylquinoline-4-carboxamide

1.75 g (3.2 mmol) of (S)-N-(α-ethylbenzyl)-3-[(4-benzylpiperazin-1-yl)methyl]-2-phenylquinoline-4-carboxamide (compound of Example 26) were dissolved in 50 ml of EtOH. Then, 0.5 g Pd(OH)₂ and 0.8 g (12.6 mmol) of ammonium formate were added. The mixture was heated to 80 °C and 0.12 ml (3.2 mmol) of formic acid were added. The solution was refluxed 1 hour and, after cooling, the catalyst was filtered off. The solution was evaporated *in vacuo* to dryness, dissolved in H₂O and extracted with EtOAc. The organic layer was dried over Na₂SO₄ and evaporated *in vacuo* to dryness. The crude oil was purified by flash column chromatography on 230-400 mesh silica gel, utilising a mixture of EtOAc/MeOH 90:10 containing 1 % NH₄OH (28%) as starting eluent, and a mixture of EtOAc/MeOH 80:20 containing I % NH₄OH (28%) as final eluent. The obtained oil was triturated in Et₂O to afford 1.1 g of the title compound.
C₃₀H₃₂N₄O
MW = 464.62
MP = 94-98°C [α]_{D}²⁰ = - 43.7 (c = 1; MeOH) IR: (KBr) 3400-2700, 3285, 1636, 1535, 1490, 1350, 1292, 1028, 763, 701 cm⁻¹
MS : A) ESI POS; TSQ 700; solvent: methanol/ spray 4.5 kV / skimmer: 60 V/ capillary 220 C; B) ESI DAU+465(Collision gas: Argon); *m*/*z*: A) 465 (MH+); B) 330; 287; 261; 246; 216.
- 300 MHz ¹H-NMR (DMSO-d₆):: (353 K): 8.81 (d br, 1H); 8.00 (d, 1H); 7.78-7.69 (m, 2H); 7.59-7.41 (m, 8H); 7.39 (dd, 2H); 7.29 (dd, 1H); 5.10 (dt, 1H); 3.50 (s, 2H); 2.39 (m, 4H); 2.00-1.80 (m, 6H); 0.98 (t, 3H).

### REFERENCE EXAMPLE 34: (S)-N-(α-ethylbenzyl)-3-[(3-oxo)pyrrolidin-1-ylmethyl]-2-phenylquinoline-4-carboxamide

0.075 ml (0.86 mmol) of oxalyl chloride were dissolved, under nitrogen atmosphere, in 2 ml of dry CH₂Cl₂. The solution was cooled to -55°C and 0.135 ml (1.9 mmol) of DMSO, dissolved in I ml of dry CH₂Cl₂ were added dropwise, keeping the temperature at -55°C. The solution was maintained under stirring for 2 minutes, then 0.4 g (0.86 mmol) of (S)-N-(α-ethylbenzyl)-3-[(3-(S)-hydroxy)pyrrolidin-1-ylmethyl]-2-phenylquinoline-4-carboxamide (compound of Example 32) dissolved in 2 ml of dry CH₂Cl₂ were added dropwise, maintaining the temperature between -55 and -50°C. After 15 minutes, 0.56 ml (4.0 mmol) of TEA were added dropwise and the temperature was allowed to raise to room temperature. 5 ml of H₂O were added, the organic layer was separated and washed with sat. sol. NaCl, dried over Na₂SO₄, filtered and evaporated *in-vacuo* to dryness. The residue was purified by gradient flash column chromatography on 230-400 mesh silica gel using as starting eluent a mixture of hexane/EtOAc 70:30 and as final eluent hexane/EtOAc 50:50. The crude product was triturated with pentane/*i*-Pr₂O to yield 0.25 g of the title compound.
C₃₀H₂₉N₃O₂
M.P. = 142-145°C
M.W. = 463.58
[α]_{D}²⁰ = -50.4 (c=0.5, MeOH)

The compounds of the Examples are summarised in Tables 1 and IA, while in Table 2 spectroscopic data of compounds of Examples 4-16, 19-27, 29-43 are reported.

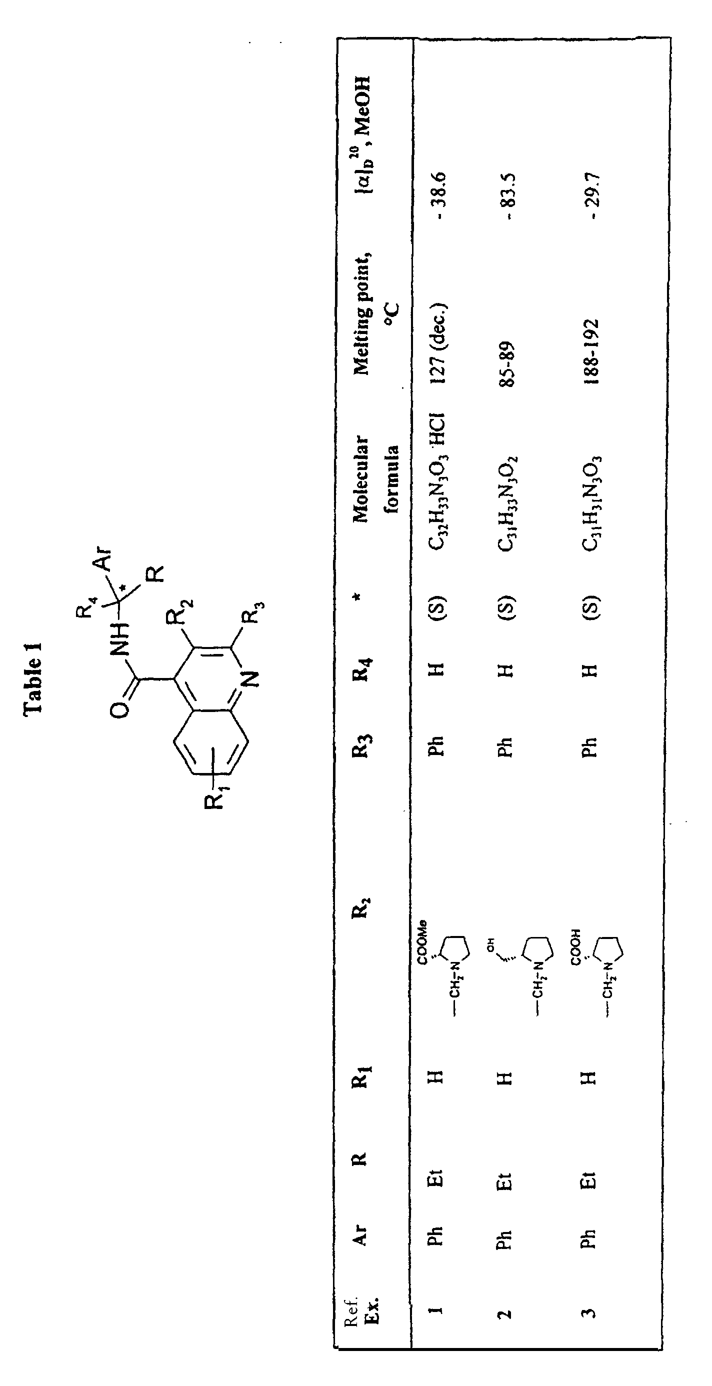

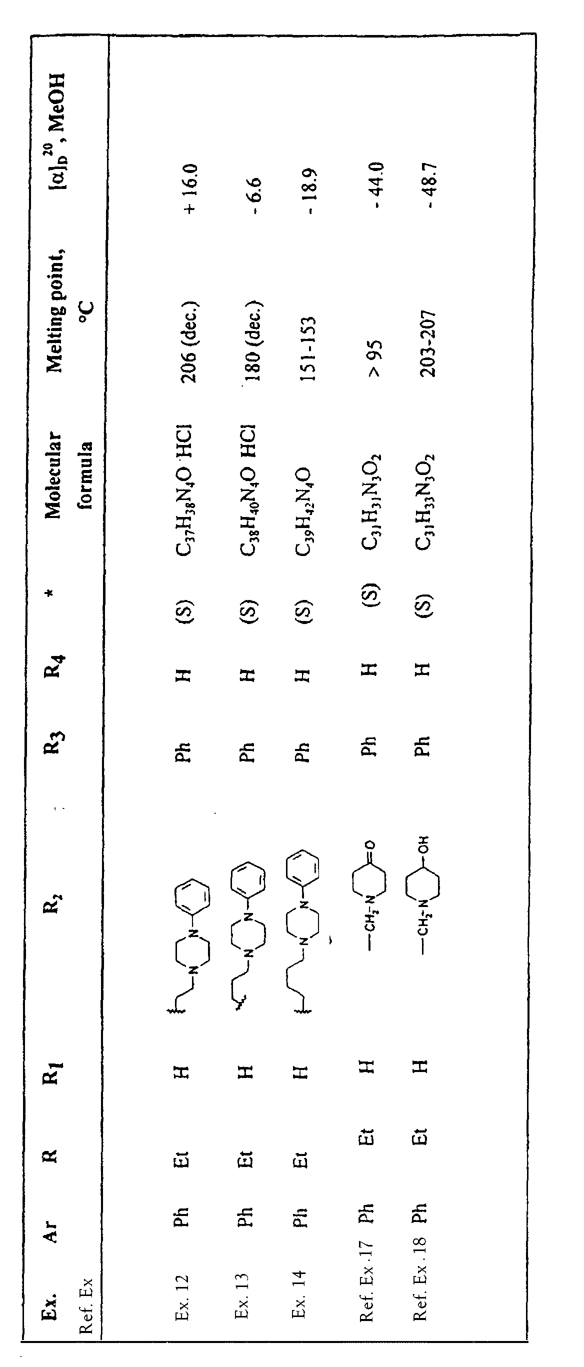

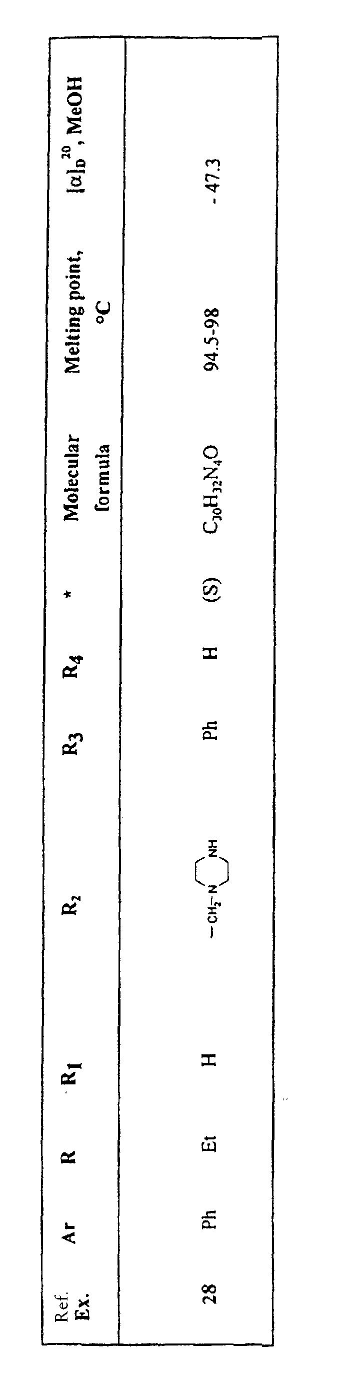

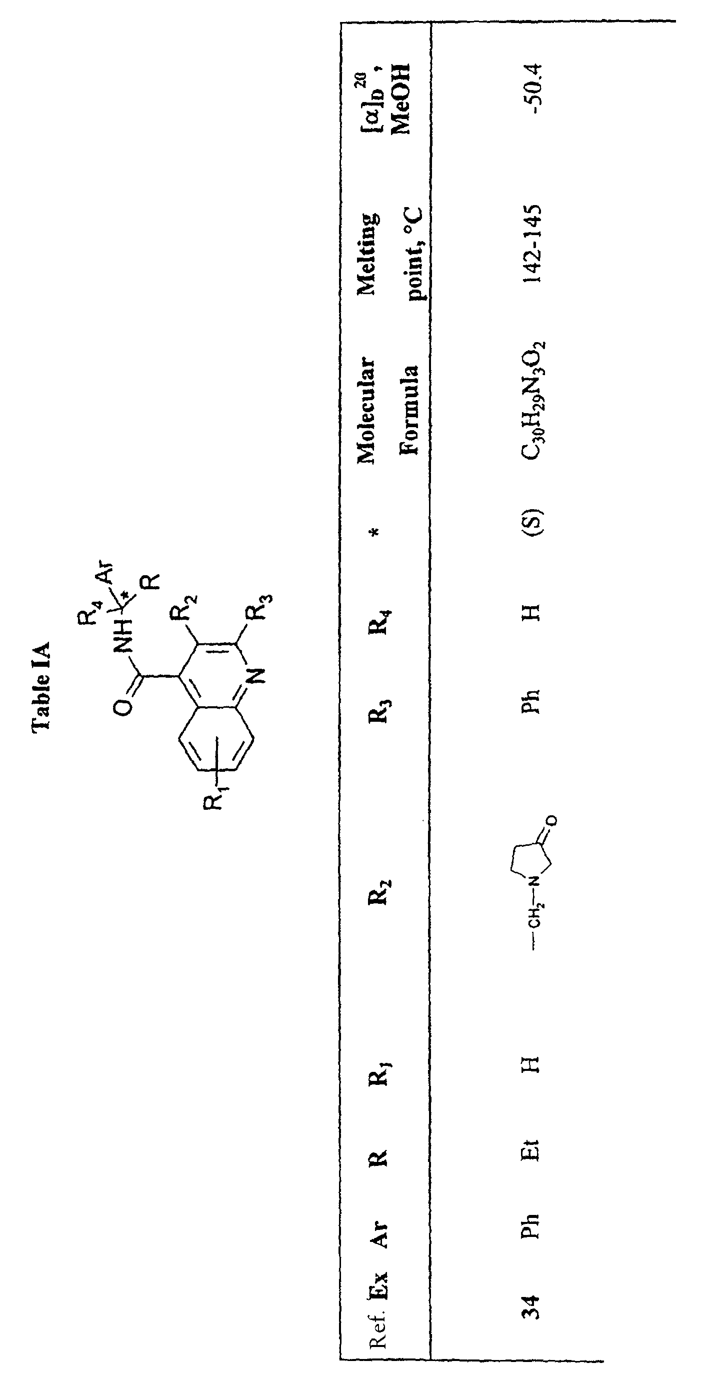

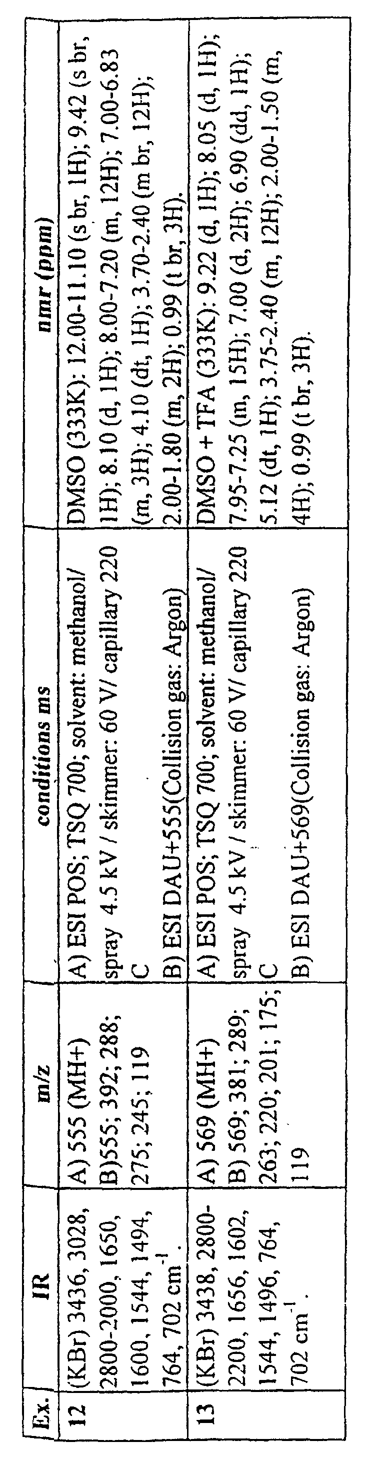

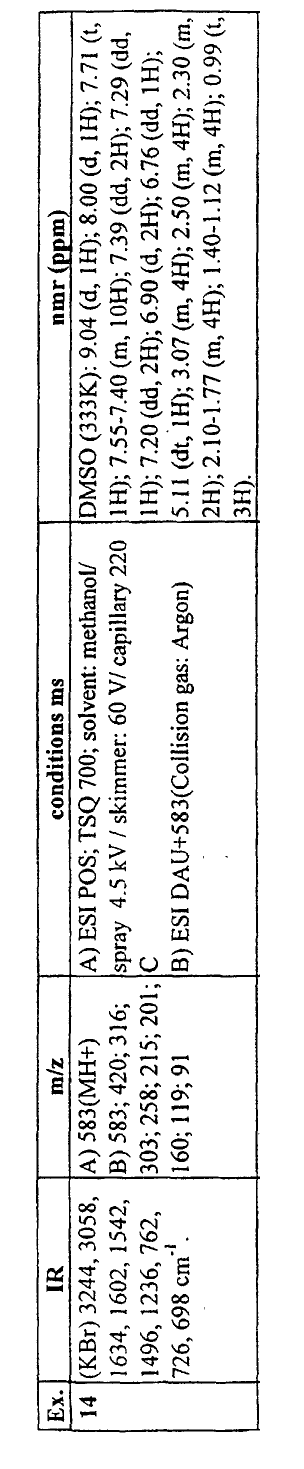

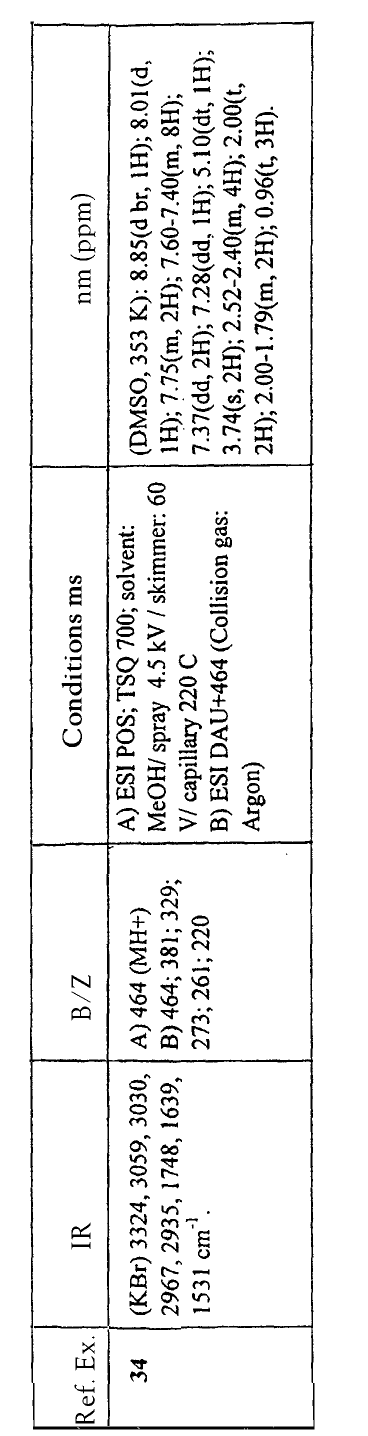

**Table III**

| **(Pharmacological data** | | |
|---|---|---|
| **Ex. No.** | **Binding affinity, Ki (nM)** | |
| | **hNK-3-CHO**^{***a***} | **hNK-2-CHO**^{***b***} |
| Ref. Ex. 2 | 1.9 | 67.9 |
| 11 | 4.0 | 66.8 |
| Ex. 12 | 1.9 | 39.0 |
| Ex. 13 | 0.3 | 3.8 |
| Ex. 14 | 1.1 | 22.2 |
| Ref. Ex. 17 | 1.5 | 42.7 |
| Ref.Ex. 18 | 2.3 | 44.6 |
| Ref. Ex. 34 | 1.5 | 38.0 |
| hNK-3-CHO^{*a*} = human neurokinin-3 receptor expressed in CHO cell lines. | | |
| hNK-2-CHO^{*b*} = human neurokinin-2 receptor expressed in CHO cell lines. | | |

## Claims

1. A compound of formula (I), or a salt or solvate thereof, 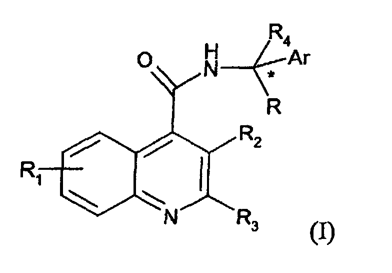 wherein, R is ethyl, R₁ is H, R₃ is phenyl, R₄ is H, Ar is phenyl and R₂ is selected from the following substituents:

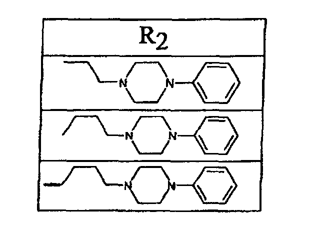

2. A compound of formula (I), according to claim 1, wherein R is ethyl, R₁ is H, R₃ is phenyl, R₄ is H, Ar is phenyl and R₂ is

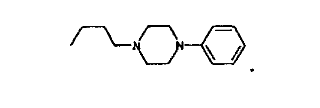

3. A process for the preparation of a compound of formula (I), or a salt thereof and/or a solvate thereof, which process comprises reacting a compound of formula (II) or an active derivative thereof: 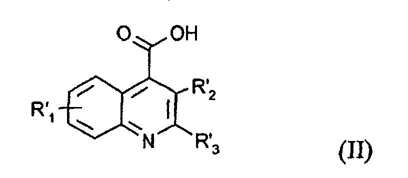 wherein R'₁, R'₂ and R'₃ are R₁, R₂ and R₃ respectively as defined in relation to formula (I) or a group convertible to R₁, R₂ and R₃ respectively; with a compound of formula (III): 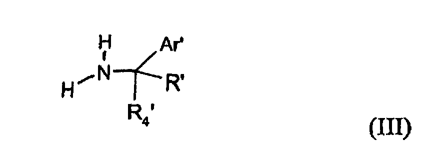 wherein R', R₄' and Ar' are R, R₄ and Ar as defined for formula (I) or a group or atom convertible to R, R₄ and Ar respectively; to form a compound of formula (Ib): 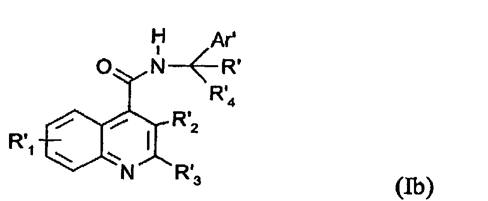 wherein Ar', R', R'₁, R'₂, R'₃ and R'₄ are as defined above, and thereafter carrying out one or more of the following optional steps:
(i) converting any one of Ar', R', R'₁, R'₂, R'₃ and R'₄ to Ar, R, R₁, R₂, R₃ or R₄ respectively as required, to obtain a compound of formula (I);
(ii) converting a compound of formula (I) into another compound of formula (I); and
(iii) preparing a salt of the compound of formula (I) and/or a solvate thereof.

4. A pharmaceutical composition comprising a compound of formula (I) according to claim 1 or claim 2, or a pharmaceutically acceptable salt or solvate thereof, and a pharmaceutically acceptable carrier.

5. A compound of formula (I) according to claim 1 or claim 2, or a pharmaceutically acceptable salt or solvate thereof, for use as an active therapeutic substance.

6. A compound of formula (I) according to claim 1 or claim 2, or a pharmaceutically acceptable salt or solvate thereof, for the treatment or prophylaxis of the Primary and Secondary Conditions.

7. The use of a compound of formula (I) according to claim 1 or claim 2, or a pharmaceutically acceptable salt or solvate thereof, in the manufacture of a medicament for the treatment of the Primary and Secondary Conditions.

8. A compound of formula (I) according to claim 1 or claim 2, for use as a diagnostic tool for assessing the degree to which neurokinin-3 and neurokinin-2 receptor activity (normal, overactivity or underactivity) is implicated in a patient's symptoms.

## Patentansprüche

1. Verbindung der Formel (I) oder ein Salz oder Solvat davon, 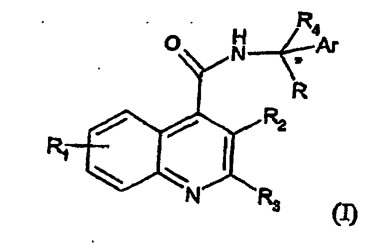 wobei R eine Ethylgruppe ist, R₁ H ist, R₃ eine Phenylgruppe ist, R₄ H ist, Ar eine Phenylgruppe ist und R₂ aus nachstehenden Substituenten ausgewählt ist:

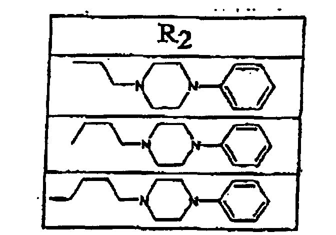

2. Verbindung der Formel (I) gemäß Anspruch 1, wobei R eine Ethylgruppe ist, R₁ H ist, R₃ eine Phenylgruppe ist, R₄ H ist, Ar eine Phenylgruppe ist und R₂ 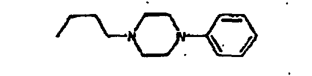 ist.

3. Verfahren zur Herstellung einer Verbindung der Formel (I) oder eines Salzes und/oder eines Solvates davon, wobei das Verfahren umfasst Umsetzen einer Verbindung der Formel (II) oder eines wirksamen Derivats davon: 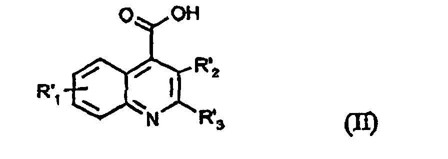 wobei R'₁, R'₂ und R'₃ R₁, R₂ beziehungsweise R₃ ist, wie in bezug auf Formel (I) definiert oder ein in R₁, R₂, beziehungsweise R₃ urwandelbarer Rest; mit einer Verbindung der Formel (III) 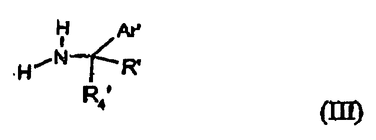 wobei R', R₄' und Ar' R, R₄ und Ar ist, wie in bezug auf Formel (I) definiert oder ein Rest oder Atom, das in R, R₄ beziehungsweise Ax umwandelbar ist; um eine Verbindung der Formel (Ib) zu bilden: 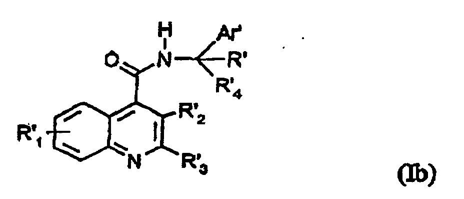 wobei Ar', R', R'₁, R'₂, R'₃ und R'₄ wie vorstehend definiert sind, und nachfolgend Durchführen eines oder mehrerer der nachstehenden optionalen Schritte:
(i) Umwandeln eines Restes aus Ar', R', R'₁, R'₂, R'₃ und R'₄ in Ar, R, R₁, R₂, R₃ beziehungsweise R₄, falls notwendig, um eine Verbindung der Formel (I) zu erhalten;
(ii) Umwandeln einer Verbindung der Formel (I) in eine andere Verbindung der Formel (I); und
(iii) Herstellen eines Satzes der Verbindung der Formel (I) und/oder eines Solvates davon.

4. Arzneimittel, umfassend eine Verbindung der Formel (I) nach Anspruch 1 oder Anspruch 2 oder ein pharmazeutisch verträgliches Salz oder Solvat davon und einen pharmazeutisch verträglichen Träger.

5. Verbindung der Formel (I) nach Anspruch 1 oder Anspruch 2 oder ein pharmazeutisch verträgliches Salz oder Solvat davon zur Verwendung als therapeutischer Wirkstoff.

6. Verbindung der Formel (I) nach Anspruch 1 oder Anspruch 2 oder ein pharmazeutisch verträgliches Salz oder Solvat davon zur Behandlung oder Prophylaxe von primären und sekundären Zuständen.

7. Verwendung einer Verbindung der Formel (I) nach Anspruch 1 oder Anspruch 2 oder eines pharmazeutisch verträglichen Salzes oder Solvates davon zur Herstellung eines Medikaments zur Behandlung von primären und sekundären Zuständen.

8. Verbindung der Formel (I) nach Anspruch 1 oder Anspruch 2 zur Verwendung als diagnostisches Hilfsmittel zur Bestimmung inwieweit Neurokinin-3- und Neurokinin-2-Rezeptoraktivität (normal, überaktiv, unteraktiv) mit den Symptomen eines Patienten in Zusammenhang steht.

## Revendications

1. Composé de formule (I), ou un de ses sels ou produits de solvatation 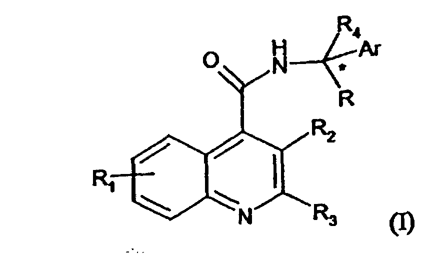 formule dans laquelle R représente un groupe éthyle, R₁ représente H, R₃ représente un groupe phényle, R₄ représente H, Ar représente un groupe phényle et R₂ est choisi parmi les substituants suivants :

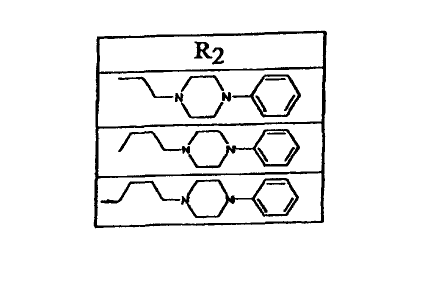

2. Composé de formule (I) suivant la revendication 1, dans lequel R représente un groupe éthyle, R₁ représente H, R₃ représente un groupe phényle, R₄ représente H, Ar représente un groupe phényle et R₂ représente

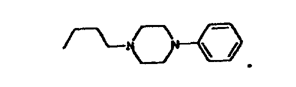

3. Procédé pour la préparation d'un composé de formule (I) ou d'un de ses sels et/ou d'un de ses produits de solvatation, procédé qui comprend la réaction d'un composé de formule (II) ou d'un de ses dérivés actifs : 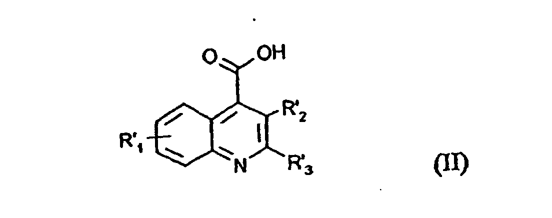 formule dans laquelle R'₁, R'₂ et R'₃ représentent respectivement des groupes R₁, R₂ et R₃ répondant aux définitions mentionnées en rapport avec la formule (I) ou des groupes pouvant être convertis respectivement en R₁, R₂ et R₃ ; avec un composé de formule (III) : 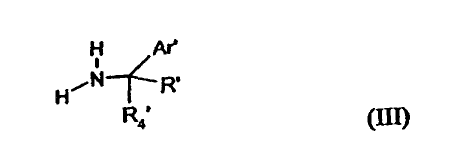 dans laquelle R', R₄' et Ar' représentent des groupes R, R₄ et Ar répondant aux définitions mentionnées par la formule (I) ou des groupes ou atomes pouvant être convertis respectivement en R, R₄ et Ar ; pour la formation d'un composé de formule (Ib) : 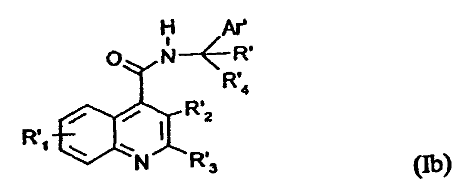 dans laquelle Ar', R',R'₁, R'₂, R'₃ et R'₄ répondent aux définitions précitées, et ensuite la mise en oeuvre d'une ou plusieurs des étapes facultatives suivantes :
(i) conversion de l'un quelconque de Ar', R', R'₁, R'₂, R'₃ et R'₄ en Ar, R, R₁, R₂, R₃ ou R₄, respectivement, de la manière requise, pour obtenir un composé de formule (I) ;
(ii) conversion d'un composé de formule (I) en un autre composé de formule (I) ; et
(iii) préparation d'un sel du composé de formule (I) et/ou d'un de ses produits de solvatation.

4. Composition pharmaceutique comprenant un composé de formule (I) suivant la revendication 1 ou la revendication 2, ou un de ses sels ou produits de solvatation pharmaceutiquement acceptables, et un support pharmaceutiquement acceptable.

5. Composé de formule (I) suivant la revendication 1 ou la revendication 2, ou un de ses sels ou produits de solvatation pharmaceutiquement acceptables, destiné à être utilisé comme substance thérapeutique active.

6. Composé de formule (I) suivant la revendication 1 ou la revendication 2, ou un de ses sels ou produits de solvatation pharmaceutiquement acceptables, pour le traitement ou la prophylaxie des Affections Primaires et Secondaires.

7. Utilisation d'un composé de formule (I) suivant la revendication 1 ou la revendication 2, ou d'un de ses sels ou produits de solvatation pharmaceutiquement acceptables, dans la production d'un médicament destiné au traitement des Affections Primaires et Secondaires.

8. Composé de formule (I) suivant la revendication 1 ou la revendication 2, destiné à être utilisé comme outil de diagnostic pour évaluer le degré auquel l'activité de récepteur de neurokinine-3 et neurokinine-2 (normale, suractivité ou sous-activité) est impliquée dans les symptômes d'un patient.
